# EUROPEAN PATENT SPECIFICATION

(11) **EP 3 357 541 B1**
(45) Date of publication and mention of the grant of the patent: **31.03.2021**
(21) Application number: 17425013.4
(22) Date of filing: 06.02.2017
(51) Int. Cl.: A61P 39/02, A61K 9/00, A61K 31/10, A61K 31/375, A61K 31/51, A61K 33/30

(54) **METHYLSULFONYLMETHANE COMPOSITION FOR REDUCING BLOOD ALCOHOL CONTENT**
METHYLSULFONYLMETHAN-ZUSAMMENSETZUNG ZUR VERRINGERUNG DES BLUTALKOHOLGEHALTS
COMPOSITION DU MÉTHYLSULFONYLMÉTHANE POUR DIMINUER LA TENEUR EN ALCOOL DANS LE SANG

(43) Date of publication of application: 08.08.2018
(73) Proprietor: Italfarmacia S.r.l., 00155 Roma (IT)
(72) Inventor: GIAMMARTINI, Enrico, 00136 Rome (IT); MARCHETTI, Mario, 00155 Rome (IT); MARCHETTI, Marco, 00155 Rome (IT); MARCHETTI, Massimiliano, 00155 Rome (IT); DE LORENZO, Antonino, 00133 Rome (IT); DI RENZO, Laura, 00133 Rome (IT)
(74) Representative: Sarpi, Maurizio

(56) References cited:
- US-A1- 2004 082 667
- George L Floersheim: "Protection against acute ethanol toxicity in mice by zinc aspartate, glycols, levulose and pyritinol", Agents and Actions, 1 January 1985 (1985-01-01), pages 580-584, XP055601178, Retrieved from the Internet: URL:https://pillbuys.com/research/Pyritino l/3.pdf [retrieved on 2019-07-01]

## Description

### Technical field

The present invention relates to a composition and method for the treatment of alcohol hangover disturbances resulting from excessive alcohol ingestion and subsequent ethanol metabolism, and is particularly directed to a method which comprises administering a composition in an amount sufficient to antagonize the central nervous system depressant effects of ethanol when administered to animals, and in particular, to mammals intoxicated or narcotized with ethanol.

### Background of the invention

Ethanol, or ethyl alcohol, is a product of the fermentative process and constitutes the active pharmacologic ingredient of beverages such as beer, wine and distilled spirits. The alcohol-containing beverages are widely used in most of the world's societies for their euphoric properties, to enhance the enjoyment of meals, and as social lubricants. Their use has been ingrained, particularly in Western societies, since pre-historic times, and the effects of alcohol, both positive and pathologic, have been recorded in the history and literature of every society and of every age. However, only during the last two to three decades science has taken up the challenge of trying to understand the mechanisms by which alcohol produces both its pleasurable as well as its damaging effects.

In particular, alcohol, through its metabolite, acetaldehyde, interferes with the maintenance of proper circulating levels of pyridoxal phosphate, the active form of vitamin B6 and essential for the conversion of methionine to cysteine. Cysteine, in turn, is important for the protection of the body from the damage due to peroxides, free radicals and the aldehydes produced from ethanol or derived from other sources.

Ethyl alcohol taken orally is rapidly absorbed by the stomach and first part of the intestine. Alcohol from the digestive tube is distributed by a simple diffusion mechanism in all body fluids and tissues, and mainly accumulates in the nervous tissue. The immediate effects of alcohol will therefore occur mainly in the brain, partly because its distribution is focusing initially on the most vascularized organs and from these diffuse only later to other body districts, such as the skeletal muscles. Subsequently, however, a distribution equilibrium is reached, so that in the brain the alcohol concentration decreases and simultaneously, and consequently, the psychic disorders decrease as well. It takes from 2 to 6 hours to complete the absorption process, depending on factors such as the presence of food and other liquids, the time taken for the ingestion of the alcoholic drink, the biological variability between individuals. The alcohol, due to its high solubility in water and low molecular weight, just absorbed is rapidly distributed in tissues, wherein it cannot be stored, and fluids of the body, even passing through the brain and the placental barrier. The maximum plasma concentration is reached 20 minutes after intake; saliva and breath closely follow the changes of blood alcohol, while urine reaches its maximum alcohol concentration about two hours later.

Urine and exhaled air are also the main routes of elimination of alcohol and oxidation products thereof.

After absorption, the ethyl alcohol is almost completely metabolized (90-98%) by oxidative route, at a speed directly proportional to body weight and constant in time. In humans, the liver is responsible for 80% of alcohol methabolism, and it can oxidizes 2 mmols of alcohol per gram of tissue per minute, corresponding to 92 mg of ethanol. The most important reaction of alcohol metabolism takes place by action of the enzyme alcohol dehydrogenase which catalizes oxidation of NAD (nicotinamide adenine dinucleotide) acting as coenzyme and hydrogen acceptor, the reaction produces acetaldehyde according to the reaction scheme:

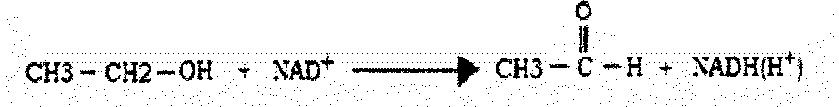

Alcohol dehydrogenases (ADH) belong a group of dehydrogenase enzymes occurring in many organisms and facilitating the interconversion between alcohols and aldehydes or ketones with the reduction of nicotinamide adenine dinucleotide (NAD+ to NADH).

Alcohol dehydrogenase has a dimeric structure containing 4 atoms of Zn for molecule; two of these atoms are located in correspondence of the active site and contribute to the binding of the substrates (NAD+ and ethanol) to the enzyme, the other two have the function of stabilizing the tertiary structure. The dependence of ADH by Zn is consistent with the increased need for Zn in chronic alcoholics. Alcohol dehydrogenase is present in the human liver as various isoforms, having slightly different properties from each other. The class I isozyme (ADH1) catalyzes the rate limiting step of oxidative pathway of ethanol metabolism in the liver [Edenberg H.J. Alcohol Res. Health 2007, 30: 5-13]. Furthermore, the enzyme is expressed in the stomach lining and even more in the liver lining cells. The enzyme varies between men and women, between young and old, and between populations from different geographical areas. European populations (Caucasian) have two major isoenzymes (cytosolic and mitochondrial) and are able to metabolize alcohol to a greater extent than Asian or American people, wherein about 50% of individuals have only the first isozyme.

In the non-high intake of ethanol condition, the enzyme alcohol dehydrogenase is solely responsible for the alcohol catabolism, while for high blood alcohol levels other enzyme systems are involved, such as liver catalase. The contribution of liver catalase in ethanol oxidation is meaningful only when ethanol is present in relatively high concentrations, and catalyzes the chemical reaction:

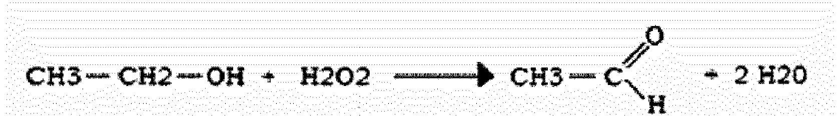

Thus, liver catalase produces acetaldehyde and consumes hydrogen peroxide, determining a protective function against peroxidation.

In the liver there is also a microsomal system oxidizing ethanol (MEOS), which consists of certain mixed function of oxidases associated to the smooth hepatocyte endoplasmic reticulum, which essentially is part of the P-450 cytochrome system.

The catalyzed reaction is:

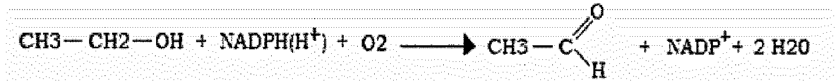

Similarly to other mixed function involved in the detoxification of drugs, MEOS has a lower affinity for methanol than alcohol dehydrogenase, however, it can accelerate the action of the latter by removing the formed NADH by trans-hydrogenation, as the MEOS requires NADPH as co-enzyme.

When continuous and plenty consumption of alcohol occurs, as in chronic alcoholics, and the liver alcohol concentration elevates, the MEOS system increases the activity. Moreover, all the conditions favoring the reoxidation of NADH and the factors inducing the cytochrome P-450 system, such as some pharmacological treatments, corticosteroids, chronic alcohol intake, cause a further increase in the rate of ethanol oxidation.

There are also other routes of elimination of ethanol, for example, very small amounts of ethanol are conjugated with glucuronic acid, through a microsomal glucoronyl-transferasi.

Non-modified ethyl alcohol is excreted in not more than 2% of the intaken amount through kidneys and lungs. Small amounts of ethanol are also found in the saliva and other body fluids; in the event of massive ingestion it can rise up to 10%.

Some authors highlight a non-oxidative metabolic pathway, but the mechanism thereof is not entirelydisclosed.

The rate of alcohol metabolism has been calculated in 10 ml per hour, but this value can also be influenced by the intaken amount, as a higher dose is oxidized proportionally more quickly than a lower dose, for example, 33 ml of alcohol (corresponding to one third of liter of wine) requires four hours to be completely oxidized, whereas a three times higher dose, i.e. equal to 100 ml of alcohol (corresponding to a liter of wine) requires only 9 hours.

It has been calculated that the maximum amount of alcohol that can be metabolized by a subject within 24 hours is 380 ml, equal to about 4 liters of wine or 1 liter of whiskey or brandy, but of course these are exceptional situations, such as in acute alcoholism, as in the case of daily chronic alcoholism, a normal liver, but evidently already subjected to a detoxification overwork, metabolizes not more than 160 ml of alcohol in 24 hours.

It is important to note that the rate of oxidation can be increased by a greater availability of the enzyme alcohol dehydrogenase, which can be congenital or acquired. Usually large size individuals have a better tolerance to alcohol, probably because the alcohol dehydrogenase, mainly concentrated in the liver, is present in higher amounts due to the proportional increase in liver volume.

The first product yielded in the oxidation of ethanol is the acetic aldehyde, which is an even more toxic substance than ethyl alcohol and probably is also carcinogenic. The discomfort resulting from alcohol hangover are caused by acetic aldehyde, however, such illnesses are due also to the presence of other substances contained in beverages which are high in spirits, or the anti-fermentative agents present mainly in white and dessert wines, such as bisulfites.

In order to quickly eliminate acetaldehyde the body resorts to another enzyme: aldehyde dehydrogenase.

Aldehyde dehydrogenase is a polymorphic enzyme responsible for the oxidation of aldehydes to carboxylic acids. These polymorphic enzymes are present in many tissues of the body, but the highest concentration is in the liver. The reaction catalyzed by aldehyde dehydrogenase is:

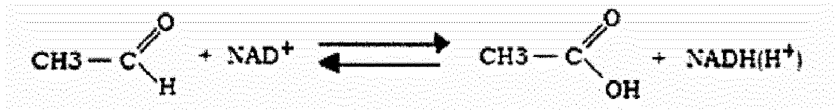

The reaction is virtually irreversible because of the very low affinity of acetate for the enzyme, which instead has a high affinity for the aldehyde. The aldehyde dehydrogenase plays a crucial role in maintaining low blood levels of acetaldehyde during the oxidation of alcohol.

The use of various compounds to contrast the toxic effects of alcohol is demonstrated in a number of patents. For example, US. Pat. No. 4,528,295 discloses the use of methionine to reduce the blood acetaldehyde level (acetaldehyde being a harmful metabolite of alcohol ingestion) by ingestion of methionine prior to drinking alcohol.

U.S. Pat. No. 3,860,719 claims that 2-[(3,4-dichlorophenoxy)methy]-2-imidazoline or a pharmaceutically acceptable salt thereof, is effective in combating ethanol intoxication in mammals when internally administered. Many other compounds have been claimed effective for this purpose.

According to EP0264430 the oral administration of activated charcoal significantly reduces the blood alcohol concentration in a drinking person.

Antonelli F. et al. reports the experimental data on a mixture of vitamins and antioxidant that shows its action in reducing the rate of breath alcohol levels in healthy subjects. The main components of the mixture are as follows: 500 mg of vitamin C, 56 mg of vitamin B5, 56 mg of vitamin D, 3.3 mg of vitamin B9, 222 mg of pyruvic acid, 120 mg of citric acid, 250 mg of tartaric acid and 77.8 g of carbohydrates [Antonelli F. Reduction of breath alcohol levels in healthy subjects by cytoethyl nternational, Journal of Nutrition and Food Sciences, 2013; 2: 196-199].

Furthermore, in the market several products promoting alcohol metabolism are available.

For example, a product containing plant extracts used in the traditional Asian medicine has been developed to protect the body against side effects of alcohol by fast excretion of acetaldehyde. Substances contained in the extracts accelerate degradation of alcohol, protect the liver from intoxication by toxic metabolites and exert a significant anti-oxidative effect. Constituents of this product are: fruits of the grapevine (*Vitis vinifera*), especially its epidermis, containing biologically active polyphenolic compounds with remarkable anti-oxidative and anti-radical effects, *Phyllatanthus amarus,* a frequent constituent of the traditional medicine of India, containing primarily the hydrolyzable tannin amarin, fruits of *Phyllanthus emblica,* fruits of the date palm, tops of *Andrographis paniculata,* and endive.

However, the use of this product is not risk free, as the medicinal use of some of the constituents has a potential adverse effect. Experimental observations indicate that in the treated groups rats that have taken in particular *Phyllatanthus amarus* showed some varying degree of distortion and disruption in microanatomy of the kidney, including interstitial oedema and tubular necrosis, when compared to the control group. [Josiah Obaghwarhieywo Adjene and Ezekiel Uba Nwose Histological effects of chronic administration of Phyllanthus amarus on the kidney of adult Wistar rat. N Am J Med Sci. 2010 Apr; 2: 193-195] .

At the same purpose it is available also a combination of active principles consisting of liver hydrolyzate, amino acids, niacin, vitamin B12, choline, inositol and cysteine. The product claims to support the liver in the elimination of drugs, toxic substances and metabolism products, to prevent the formation of acetaldehyde, to promotes detoxification after excessive alcohol consumption, to prevent the stomach heaviness feeling and discomfort after the intake of high-fat foods; furthermore it is supposed to contribute to an improvement of disturbances in numerous diseases of the liver. Therefore, this product has not a specific activity on alcohol metabolism, but rather it exerts multiple effects, and it seems to be useful for several unrelated liver disorders and to alleviate non-specific symptoms.

A further product consisting of granules containing a mixture of glucose monohydrate, glicinum and sodium formiate is indicated for prevention of alcohol intoxication; it reduces symptoms of alcohol intoxication, increases the functional activity of the cerebral cortex, has positive effect on liver function, improves general well-being. However, the product is more suitable for treating chronic alcoholism than alleviating hangover symptoms, as one of the reported mechanisms of its action is normalizing the catecholamine changes caused by the ethanol intoxication in tissues.

US2004082667 A1 discloses a method of treatment and prevention of alcohol-induced hangover by administering 0.06 onces of substantially pure methylsulfonylmethane in powdered form mixed with approximatively 8 onces of water or juice and administered to a person experiecing the symptoms of alcohol-induced hangover. Alternatively the therapeutically effective amount of methylsulfonilmethane is incorpored into a solid oral dosage form, such as a tablet or capsule and administered to a person experiencing the symptoms of alcohol-induced hangover.

Therefore, it is evident that it is desirable to have alternative means of simply, safely and, moreover, more quickly reducing the alcohol concentration in the blood of a person who has consumed sufficient alcohol to become intoxicated than the solutions available in the prior art.

### Summary of the invention

The technical problem of having alternative means of simply, safely and more quickly reducing the blood alcohol level is solved by the present invention. An useful alternative is disclosed by providing an effective composition for reducing the concentration of alcohol in the blood by increasing the alcohol metabolism, so as to sensibly reduce the unpleasant consequences related to excessive alcohol intake and hangover, and the relative method of use as herein disclosed.

The scope of the present invention is defined in the claims; any references in the description to methods of treatment refer to the compositions of the present invention for use in a method for treatment by therapy.

### Detailed description of the invention

It is an object of the present invention to provide a composition for ameliorating symptoms following the excessive intake of alcohol, for reducing the blood concentration of alcohol in a subject who has taken on an amount of alcohol as to cause toxicity, for more quickly recovering from alcohol hangover disturbances.

It is another object of the present invention the use of said composition for the treatment of alcohol hangover disturbances resulting from excessive alcohol ingestion and the subsequent ethanol metabolism.

After ingestion of large amounts of alcohol, acetaldehyde levels increase in the liver and subsequently in the circulation. Circulating acetaldehyde can produce a stress-like response by releasing norepinephrine and epinephrine from adrenals, cause facial flushing by dilating peripheral blood vessels, and cause headaches, nausea and vomiting, and a number of other symptoms associated with the ill effects of ingestion of large amounts of alcohol. The presence of high levels of acetaldehyde in the blood, liver, and other organs also tends to slow alcohol metabolism. However, if acetaldehyde is somehow inactivated, or eliminated, all of the above mentioned adverse physiologic effects produced by acetaldehhyde can be diminished

Surprisingly, it has been observed that a composition comprising methylsulfonylmethane, MSM, is effective in reducing the concentration of alcohol in a subject that has taken on an amount of alcohol as to cause toxicity, and sensibly reduces the unpleasant consequences related to excessive alcohol intake and hangover.

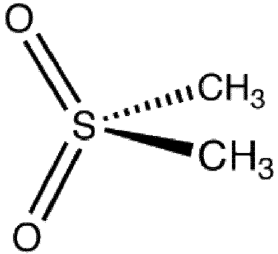

MSM is an organosulfur compound having formula (CH₃)₂SO₂. MSM is structurally related to dimethyl sulfoxide being the oxidized form thereof, when ingested, DMSO is converted into MSM, but the behavior of these two compounds is different. DMSO is a highly polar solvent and an excellent ligand, with water-like dissolving properties, whereas MSM is less polar and less reactive. MSM is well known as a nutritional supplement and as a pharmaceutical agent [Jacob and Appleton, MSM; the definitive guide: A comprehensive review of the science and therapeutics of Methylsulfonylmethane, Topanga, Calif.: Freedom Press, 2003]. MSM is also known to be useful for the treatment of osteoarthritis [Kim et al., Osteoarthritis Cartilage, 2006; 14: 286-94] and hay fever [Barrager et al., J. Altern. Complement. Med., 2002; 8: 167-74]. One of skill in the relevant art will be familiar with these uses.

MSM is highly water soluble. At room temperature, aqueous solutions of 20% MSM can be easily prepared. Aqueous solutions of higher MSM concentrations are possible at temperatures above room temperature.

The MSM is naturally present in many plant foods and Equisetum plant. For its chemical properties and some biological activity, MSM is currently used in the common integrative practice.

The nutraceutical properties of methylsulfonylmethane derive from its content of sulfur in bioavailable form, thanks to the presence of an organic component which facilitates absorption thereof.

It is known that sulfur is an essential component of living cells and represents the seventh or eighth most abundant element in the human body in terms of weight, comparable in this respect to potassium and slightly more abundant than sodium and chloride.

In a 70 kg adult human organism we find about 140 grams of sulfur, especially concentrated in muscle proteins, in some coenzymes (e.g. glutathione, alpha-lipoic acid, coenzyme A), in some hormones (e.g. insulin), in glycophospholipids of nerve tissue, in some vitamins (thiamine and biotin), in glycosaminoglycans, such as chondroitin sulfate of the articular cartilage.

The disulfide bonds (S-S) are extremely important in protein structures, which gives toughness and strength.

For these reasons, the MSM is commonly used for its anti-inflammatory properties, for the chondroprotective properties as it seems to enhance the synthesis of articular cartilage, it is believed that the integration of methylsulfonylmethane can help reducing pain and inflammation, increasing the mobility of arthritic and inhibiting further cartilaginous damages, increase skin and hair tropism, and promoting wound healing, normalizing gastrointestinal functions.

Among the many useful properties attributed to the methylsulfonylmethane, surprisingly it has been found that, when included in a composition, it has also the ability to counteract the ethyl alcohol effect by stimulating the alcohol dehydrogenase and aldehyde dehydrogenase enzymes, by decreasing (halving) the time required to completely metabolize alcohol.

According to the invention the composition comprises methylsulfonylmethane in amount varying between 50 % and 70 % w/w, more preferably in amount varying between 58 % and 63 % w/w.

According to the invention the composition comprises Vitamin C in amount varying between 20 and 30% w/w, more preferably in amount varying between 23 and 26 % w/w.

The composition according to the invention comprises also other vitamins; particularly preferred in the formulation is vitamin B1, having function of amplifying the effects of other components of the composition. Vitamin B1 is in amount varying between 1,5 and 4 % w/w, more preferably in amount varying between 2 and 3 % w/w.

The composition according to the invention comprises also an organic salt of zinc, the preferred form of organic salt of zinc is zinc gluconate.

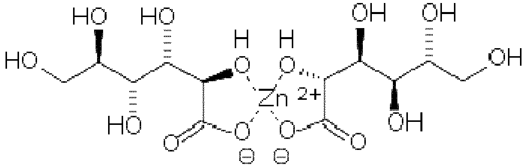

Zinc is essential for the activity of several enzymes including anhydrase, DNA polymerase, histone deacetylase, carboxypeptidase A, angiotensin converting enzyme, and alcohol dehydrogenase. Furthermore, Zn⁺⁺ is the functional group for several hydrolases. The cation activates the carbonilic group, through the formation of a coordination binding, so as to make it more sensitive to water attack. Several drugs uses the chelation of the Zn⁺⁺ in the enzyme to be inhibited.

According to the invention the organic salt of zinc is in amount varying between 1.5 and 4 % w/w, more preferably in amount varying between 2 and 3 % w/w.

The composition according to the invention may further comprise pharmaceutical acceptable excipients, such as for example, and as a non-limiting example, dispersing agents, carriers, flavoring and coloring agents. Examples of solid pharmaceutical excipients and carriers are: maize starch, magnesium stearate, talc, silicon dioxide, and the like.

According to the invention the composition for the use in the treatment of alcohol hangover disturbances can be administered orally in any convenient manner, e.g. by ingesting the solid form or an aqueous solution thereof. Preferably, the composition is administered in unit dosage form, tablets and capsules are even more preferred. It is expected that the use of tablets or capsules will make the treatment more generally available in locations where the consumption of alcohol takes place.

In a particularly preferred embodiment a dosage unit of the composition of the invention has the following exemplary formulation:

| Ingredients | Amount |
|---|---|
| Methylsulfonylmethane | 312.5 mg |
| Vitamin C | 127.5 mg |
| Vitamin B1 | 15.0 mg |
| Zinc gluconate | 12.5 mg |
| maize starch | 31.69 mg |
| magnesium stearate | 15.0 mg |
| talc | 0.16 mg |
| silicon dioxide | 0.16 mg. |

According to the present invention, the effective method for reducing the concentration of alcohol in the blood is achieved by the ingestion of a sufficient amount of composition, i.e. one, or more, dosage units as set forth above before drinking alcoholics, preferably two or three dosage units 35-45 minute prior alcohol assumption.

### Clinical study

The effectiveness of the composition according to the invention has been evaluated by the present study carried out on healthy subjects wherein ethylic alcohol metabolism has been measured.

In the study have been excluded Asian subjects, persons addicted to alcohol, obese and severely overweight subjects, suffering from atrial fibrillation patients and children under 18.

Twenty three subjects have been enrolled in the study, all subjects enrolled in the study gave their informed consent to testing.

Test was performed either fasting and after taking a minimal meal constituted of 50 g of bread and 160 ml of orange juice.

Hence, the effect of a composition comprising methylsulfonylmethane on circulating levels of acethaldehyde after administration of ethanol was monitored.

At T0 peripheral blood was harvested from tested subjects and assayed for acetaldehyde level. Then tested subjects received the composition according to the invention, 35 minutes before the alcohol intake, i.e. an amount of ethanol equal to 40 ml of grappa (alcohol 40 % vol.) in 4 minutes, (T1=T0+35'+4'). At predetermined times after the administration of ethanol, peripheral blood was harvested and assayed for acethaldehyde level by gas chromatographic technique (Tabakoff et al., Biochem. Pharmacol. 1976. 25: 1305-9) at T2=T1+10', T3=T2+10', T4=T3+15', T5=T4+17', T6=T5+5', successively measurements were performed every 5' up to the value reaches zero. Control was performed by acetaldehyde measurements in absence of composition administration.

### Results

The assay results are set forth in tables below.

**Table 1 - Control fasting test**

| T0 | T1=T0+4' | T2=T1+5' | T3=T2+5' | T4=T3+5' | T5=T4+5' | T6=T5+5' | T7=T6+5' | T8=T7+5' | T9=T8+5' |
|---|---|---|---|---|---|---|---|---|---|
| | Alcohol administration | | | | | | | | |
| | | Measurements in duplicate | | | | | | | |
| | | 1.9 | 0.7 | 0.4 | 0.3 | 0.2 | 0.1 | 0.1 | 0 |
| | | 2.0 | 0.7 | 0.5 | 0.3 | 0.2 | 0.2 | 0.1 | 0 |

**Table 2 - Composition fasting test, composition intake 35 minutes before alcohol administration**

| T0 | | T1=T0+35'+4' | T2=T1+5' | T3=T2+5' | T4=T3+5' | T5=T4+5' | T6=T5+5' | T7=T6+5' | T8=T7+5' |
|---|---|---|---|---|---|---|---|---|---|
| | Composition administration | Alcohol administration | | | | | | | |
| | | | Measurements | | | | | | |
| | 2 capsules | | 1.4 | 0.6 | 0.4 | 0.3 | 0.2 | 0.1 | 0 |
| | 2 capsules | | 1,5 | 0.7 | 0.3 | 0.2 | 0.2 | 0.1 | 0 |

**Table 3 - Composition fasting test, composition intake 45 minutes before alcohol administration**

| T0 | | T1=T0+35'+4' | T2=T1+5' | T3=T2+5' | T4=T3+5' | T5=T4+5' | T6=T5+5' | T7=T6+5' | T8=T7+5' |
|---|---|---|---|---|---|---|---|---|---|
| | Composition administration | Alcohol administration | | | | | | | |
| | | | Measurements | | | | | | |
| | 2 capsules | | 1.4 | 0.5 | 0.3 | 0.2 | 0.2 | 0.1 | 0 |

**Table 4 - Control test**

| T0 | T1=T0+4' | T2=T1+5' | T3=T2+5' | T4=T3+5' | T5=T4+5' | T6=T5+5' | T7=T6+5' | T8=T7+5' |
|---|---|---|---|---|---|---|---|---|
| | Alcohol administration | | | | | | | |
| Food intake | | Measurements in triplicate | | | | | | |
| | | 1.2 | 0.5 | 0.3 | 0.2 | 0.1 | 0.1 | 0 |
| | | 1.1 | 0.6 | 0.3 | 0.1 | 0.1 | 0.1 | 0 |
| | | 1.2 | 0.6 | 0.3 | 0.2 | 0.1 | 0.1 | 0 |

**Table 5 - Composition test, composition intake 35 minutes before alcohol administration**

| T0 | T1=T0+4' | T2=T1+5' | T3=T2+5' | T4=T3+5' | T5=T4+5' | T6=T5+5' |
|---|---|---|---|---|---|---|
| | Alcohol administration | | | | | |
| Food intake | | Measurements in duplicate | | | | |
| | 2 capsules | 1.0 | 0.5 | 0.2 | 0.1 | 0 |
| | 2 capsules | 1.1 | 0.6 | 0.3 | 0.1 | 0 |
| | 3 capsules | 0.9 | 0.4 | 0.2 | 0 | |
| | 3 capsules | 1.0 | 0.5 | 0.2 | 0 | |
| | 4 capsules | 0.7 | 0.3 | 0.3 | 0.1 | 0 |
| | 4 capsules | 0.7 | 0.3 | 0.3 | 0.1 | 0 |

By comparison of data summarized in tables 1, 2, 3, and moreover 4 and 5, the blood alcohol values decreases to zero 25 minutes following administration of 40 ml of grappa (alcohol 40 % vol.) when the subject received three capsules of the composition according to the invention, or 42 minutes following administration of 40 ml of grappa (alcohol 40 % vol.) when the subject received two or four capsules of the composition, whereas without composition the blood alcohol values decreases to zero in 62 minutes.

Therefore, the administration of the composition according to the invention resulted in significantly more rapid decreases of acetaldehyde in circulating blood levels, as alcohol is metabolized in about half of time without composition treatment.

## Claims

1. A composition for the use in the treatment of alcohol hangover disturbances resulting from excessive alcohol intake and subsequent metabolism comprising:
a) methylsulfonylmethane,
b) vitamin C,
c) an organic salt of zinc,
d) vitamin B1.

2. The composition for the use according to claim 1 wherein:
a) methylsulfonylmethane is in the range between 50 and 70 % w/w,
b) vitamin C is in the range between 20 and 30 % w/w,
c) an organic salt of zinc is in the range between 1.5 and 4.0 % w/w,
d) vitamin B1 is in the range between 1.5 and 4.0 % w/w.

3. The composition for the use according to claims 1 and 2 further comprising: pharmaceutical excipients, dispersing agents, carriers, flavoring and coloring agents.

4. The composition for the use according to claims 1 and 2 wherein:
a) methylsulfonylmethane is in the range between 58 and 63 % w/w,
b) vitamin C is in the range between 23 and 26 % w/w,
c) an organic salt of zinc is in the range between 2.0 and 3.0 % w/w,
d) vitamin B1 is in the range between 2.0 and 3.0 % w/w.

5. The composition for the use according to anyone of the preceding claims administered orally by ingesting one or more dosage units of the solid form or the aqueous solution of said composition.

6. The composition for the use according to claim 5 wherein the dosage unit has the following formulation:
| | |
|---|---|
| Methylsulfonylmethane | 312.5 mg |
| Vitamin C | 127.5 mg |
| Vitamin B1 | 15.0 mg |
| Zinc gluconate | 12.5 mg |
| maize starch | 31.69 mg |
| magnesium stearate | 15.0 mg |
| talc | 0.16 mg |
| silicon dioxide | 0.16 mg. |

7. The composition for the use according to anyone of the preceding claims wherein one, or more dosage units, are ingested 35-45 minutes prior to alcohol consumption.

8. The composition for the use according to claim 7 wherein three dosage units are ingested 35-45 minutes prior to alcohol consumption.

## Patentansprüche

1. Zusammensetzung zur Verwendung bei der Behandlung von Alkoholkaterstörungen, die von übermäßiger Alkoholaufnahme und darauffolgendem Stoffwechsel resultieren, umfassend:
a) Methylsulfonylmethan,
b) Vitamin C
c) ein organisches Salz von Zink
d) Vitamin B1.

2. Zusammensetzung zur Verwendung nach Anspruch 1, wobei:
a) Methylsulfonylmethan im Bereich zwischen 50 und 70 % Gew./Gew. liegt,
b) Vitamin C im Bereich zwischen 20 und 30 % Gew./Gew. liegt,
c) ein organisches Salz von Zink im Bereich zwischen 1,5 und 4,0 % Gew./Gew. liegt,
d) Vitamin B1 im Bereich zwischen 1,5 und 4,0 % Gew./Gew. liegt.

3. Zusammensetzung zur Verwendung nach den Ansprüchen 1 und 2, ferner: pharmazeutische Trägerstoffe, Dispergierhilfsmittel, Träger, Aromastoff und Farbstoffe umfassend.

4. Zusammensetzung zur Verwendung nach den Ansprüchen 1 und 2, wobei:
a) Methylsulfonylmethan im Bereich zwischen 58 und 63 % Gew./Gew. liegt,
b) Vitamin C im Bereich zwischen 23 und 26 % Gew./Gew. liegt,
c) ein organisches Salz von Zink im Bereich zwischen 2,0 und 3,0 % Gew./Gew. liegt,
d) Vitamin B1 im Bereich zwischen 2,0 und 3,0 % Gew./Gew. liegt.

5. Zusammensetzung zur Verwendung nach einem der vorhergehenden Ansprüche, die oral durch Einnehmen einer oder mehrerer Dosiereinheiten der festen Form oder der wässrigen Lösung der Zusammensetzung verabreicht wird.

6. Zusammensetzung zur Verwendung nach Anspruch 5, wobei die Dosiereinheit die folgende Formulierung aufweist:
| | |
|---|---|
| Methylsulfonylmethan | 312,5 mg |
| Vitamin C | 127,5 mg |
| Vitamin B1 | 15,0 mg |
| Zinkgluconat | 12,5 mg |
| Maisstärke | 31,69 mg |
| Magnesiumstearat | 15,0 mg |
| Talkum | 0,16 mg |
| Siliciumdioxid | 0,16 mg. |

7. Zusammensetzung zur Verwendung nach einem der vorhergehenden Ansprüche, wobei eine oder mehrere Dosiereinheiten 35-45 Minuten vor Alkoholkonsum eingenommen werden.

8. Zusammensetzung zur Verwendung nach Anspruch 7, wobei drei Dosiereinheiten 35-45 Minuten vor Alkoholkonsum eingenommen werden.

## Revendications

1. Composition pour son utilisation dans le traitement de troubles de xylostomiase résultant d'une absorption excessive d'alcool et du métabolisme subséquent comprenant :
a) du méthylsulfonylméthane,
b) de la vitamine C,
c) un sel organique de zinc,
d) de la vitamine B1.

2. Composition pour son utilisation selon la revendication 1 dans laquelle :
a) le méthylsulfonylméthane est présent dans la plage comprise entre 50 et 70 % p/p,
b) la vitamine C est présente dans la plage comprise entre 20 et 30 % p/p,
c) un sel organique de zinc est présent dans la plage comprise entre 1,5 et 4,0 % p/p,
d) la vitamine B1 est présente dans la plage comprise entre 1,5 et 4,0 % p/p.

3. Composition pour son utilisation selon les revendications 1 et 2 comprenant en outre : des excipients pharmaceutiques, des agents de dispersion, des vecteurs, des agents aromatisants et colorants.

4. Composition pour son utilisation selon les revendications 1 et 2 dans laquelle :
a) le méthylsulfonylméthane est présent dans la plage comprise entre 58 et 63 % p/p,
b) la vitamine C est présente dans la plage comprise entre 23 et 26 % p/p,
c) un sel organique de zinc est présent dans la plage comprise entre 2,0 et 3,0 % p/p,
d) la vitamine B1 est présente dans la plage comprise entre 2,0 et 3,0 % p/p.

5. Composition pour son utilisation selon l'une quelconque des revendications précédentes administrée par voie orale par ingestion d'une ou plusieurs unités posologiques de la forme solide ou de la solution aqueuse de ladite composition.

6. Composition pour son utilisation selon la revendication 5 dans laquelle l'unité posologique a la formulation suivante :
| | |
|---|---|
| Méthylsulfonylméthane | 312,5 mg |
| Vitamine C | 127,5 mg |
| Vitamine B1 | 15,0 mg |
| Gluconate de zinc | 12,5 mg |
| Amidon de maïs | 31,69 mg |
| Stéarate de magnésium | 15,0 mg |
| Talc | 0,16 mg |
| Dioxyde de silicium | 0,16 mg. |

7. Composition pour son utilisation selon l'une quelconque des revendications précédentes dans laquelle une, ou plusieurs unités posologiques sont ingérées 35 à 45 minutes avant la consommation d'alcool.

8. Composition pour son utilisation selon la revendication 7 dans laquelle trois unités posologiques sont ingérées 35 à 45 minutes avant la consommation d'alcool.
